# EUROPEAN PATENT SPECIFICATION

(11) **EP 3 344 150 B1**
(45) Date of publication and mention of the grant of the patent: **08.04.2020**
(21) Application number: 15797188.8
(22) Date of filing: 31.08.2015
(51) Int. Cl.: A61B 10/00

(54) **EXTRACTION AND ANALYSIS DEVICE, IN PARTICULAR FOR SYNOVIAL FLUID**
EXTRAKTIONS-UND ANALYSEGERÄT, VORALLEM FÜR SYNOVIALFLÜSSIGKEIT
DISPOSITIF D'EXTRACTION ET D'ANALYSE, EN PARTICULIER POUR LIQUIDE DE SYNOVIALE

(43) Date of publication of application: 11.07.2018
(73) Proprietor: Eon Medica S.r.l., 20052 Monza (MI) (IT)
(72) Inventor: CECCA, Emanuela Maria, 20052 Monza (IT); CULTRERI, Renza, 20052 Monza (IT); BRENICCI, Tommaso, 20052 Monza (IT)
(74) Representative: Tiburzi, Andrea
(86) International application number: PCT/IT2015/000215
(87) International publication number: WO 2017/037748

(56) References cited:
- US-A- 5 097 834
- US-A1- 2008 091 119
- US-A1- 2008 221 407
- US-A1- 2013 203 175

## Description

The present invention relates to an extraction and analysis device, in particular for synovial fluid.

More specifically, the invention relates to a device for withdrawing and in-line analysis, studied and realized in particular to allow real-time high reliability analysis of withdrawn synovial fluid, so as to have a first response for possible infection, usable in field of surgical interventions, such as for the removal of prostheses and the like, which can also be used in ambulatory or in any other situation where it is necessary to have real time diagnostic clinical results.

In the following, the description will be directed to the withdrawal and the analysis of synovial fluid, but it is clear that the same should not be considered limited to this specific use.

As it is well known at present an accurate and efficient diagnosis of periprosthetic joint infection (PJI) remains one of the most felt needs for orthopedic and microbiologists surgeons.

Several tests are commercially available for the diagnosis of articular periprosthetic infection. In recent years, many studies have been carried out to identify new markers or reagents that can contribute to a more accurate diagnosis of osteoarticular infection.

Among the potential markers or reagents of osteoarticular infection only a few have certain characteristics that make them suitable for the use in the clinical diagnostics, making as they are easily detectable in the synovial fluid through automated or easy to carry out methods.

In particular, leukocyte esterase (Leukocyte Esterase - or "LE") test is easy to carry out and easy to apply in a complex diagnostic process, which is the diagnosis of bone and joint infections.

The leukocyte esterase, also known as neutrophil elastase, or granulocyte elastase, is an enzyme synthesized by white blood cells, particularly by activated neutrophils, often found within infected body fluids.

The esterases are enzymes belonging to the class of hydrolases, which catalyze the hydrolysis reaction of the ester bond. In particular, the leukocyte esterase has the function to degrade proteins that act as a support to the connective tissue matrix.

The secretion of the enzyme leukocyte esterase by neutrophils retrieved by the infection condition, allows checking with a simple enzymatic colorimetric test the presence of an infection. This test involves, in fact, a change of the color of the pad, which depends by a hydrolytic reaction that occurs when the leukocyte esterase enzyme contacts with the chemical substances on the test pad.

The presence of granulocytic leukocytes is confirmed on the basis of the color gradation.

Furthermore, it was previously assumed (see in particular the article "Preliminary Results of a New Test for Rapid Diagnosis of Septic Arthritis with Use of Leukocyte Esterase and Glucose Reagent Strips", Mohamed Omar, Max Ettinger, Moritz Reichling, Maximilian Petri, Ralf Lichtinghagen, Daniel Guenther, Eduardo M. Suero, Michael Jagodzinski, and Christian Krettek, The Journal Of Bone And Joint Surgery, Incorporated), that the combined analysis of leukocyte esterase and glucose can improve the diagnosis of septic arthritis in the native synovial fluid, because the concentrations of leukocyte esterase are high at the site of infection, due to the increased secretion of this enzyme by neutrophils in case of inflammatory processes, while the glucose concentration is rather reduced in the case of infectious process, due to the bacterial metabolism.

In known extraction and diagnosis systems of synovial liquid a problem is that of transferring the same into vacutainers or tubes in general, in which it is highly probable contamination and loss of fluid.

In addition, a well-known problem in the field is that of allowing, at the same time, a sterile collection of the synovial liquid in a vacutainer, for it to be analyzed at a later time in the laboratory and a first test, which in real-time allows to provide both an indication of the presence of inflammation of the withdrawn synovial fluid, and a first indication of the degree of the infection. Naturally, such effects are to be obtained in a totally sterile way, avoiding any external contamination.

Further the patent application US 2008/221407 A1, US 5097834 A and US 2008/091119 A disclose an example of a device for extraction and analysis of anatomical tissues, which forms part of the state of the art.

US2013/203175 A1 discloses a device for the extraction and analysis of anatomical tissues according to the preamble of claim 1.

In light of the above, it is, therefore, object of the present invention to provide a device for real-time analysis of synovial fluid, so as to obtain a reliable diagnosis immediately.

It is also object of the present invention, to enable the use of the entire synovial liquid extracted from the patient, without losses in line and without contamination with the outside environment between the extraction of the same and the run of the test.

It is therefore specific object of the present invention device for extraction and analysis of anatomical tissues, such as synovial liquid and the like, comprising an extraction line of said tissue to be analyzed, having a needle, for the withdrawal of said tissue to be analyzed, an extraction duct, an extraction syringe, having an inlet/outlet mouth connected fluid-dynamically to said needle by means of said extraction duct, and an analysis device, fluid-dynamically connected to said inlet/outlet mouth of said extraction syringe by means of said extraction duct, said analysis device having one or more receptacles, in each of which a respective reagent is arranged, and reading means, for detecting possible alterations of said reagents.

Always according to the invention, said one or more receptacles comprise a first receptacle, into which a first reagent is placed, a second receptacle, into which a the second reagent is placed, and a third receptacle, into which a third reagent is placed.

Still according to the invention, said first reagent could be esterases, said second reagent is CRP (C-Reactive Protein) and said third reagent is glucose.

Advantageously according to the invention, said reading means could comprise a reading scale, capable to show the color variations of said reagents, allowing to detect the degree of infection of said anatomical tissue, as synovial liquid and the like.

Further according to the invention, said extraction duct of said extraction line comprises a first three-way valve, having a first port, connected to said withdrawal needle, a second port, connected to said inlet/outlet mouth of said extraction syringe, and a third port connectable to said analysis device, each of said ports being selectively opened or closed independently of the other.

Always according to the invention, said extraction line could comprise air filtering means, in their turn comprising an air filter and a respective three-way valve, having three ports selectively openable or closable independently of the other, one of said three ports being connected to said inlet/outlet mouth of said extraction syringe, and another of said three ports being connected to said air filter.

Still according to the invention, said device could comprise an anatomical tissues collecting line, having a vacutainer, fluid-dynamically connected to said inlet/outlet mouth of said suction syringe.

Advantageously according to the invention, said collecting line could comprise a suction syringe having an inlet/outlet mouth fluid-dynamically connected to said inlet/outlet mouth of said extraction syringe.

Always according to the invention, said collecting line could comprise a second three-way valve, having a first port, fluid-dynamically connected to said third port of said first three-way valve along said extraction duct of said extraction line, a second port fluid-dynamically connected to said inlet/outlet mouth of said suction syringe, and a third port, fluid-dynamically connected to said analysis device, each of said ports being selectively opened or closed independently of the other.

Still according to the invention, said collecting line could comprise filtering means comprising an air filter and a respective three-way valve, having three ports selectively openable or closable independently of the other, one of said three ports being connected to said inlet/outlet mouth of said suction syringe, and another one of said three ports being connected to said air filter.

Advantageously according to the invention, said device could comprise plasma filtering means , adapted to filter the plasma from the said tissue to be analyzed, arranged between said inlet/outlet mouth of said extraction syringe and said analysis device.

Further according to the invention, said plasma filtering means comprise a filtering syringe, having an inlet/outlet mouth, a third three-way valve, having a first port, fluid-dynamically connected to said inlet/outlet mouth of said extraction syringe, a second port, to which the inlet/outlet mouth of said filtering syringe is connected, and a third port, fluid-dynamically and selectively connected to said analysis device.

Always according to the invention, said extraction line comprise a first duct, which connects said withdrawal needle and the analysis unit, said first three-way valve being arranged along said first duct, and a second duct, which connects the extraction syringe to said first duct, a four-way valve having a first port fluid-dynamically connected to said withdrawal needle, a second port fluid-dynamically connectable to said vacutainer, a third port fluid-dynamically connectable to said extraction syringe and a fourth port, each of said ports being selectively opened or closed independently of the other, and said filtering means could comprise a filtering line, fluid-dynamically connected between said filtering syringe and said analysis unit, and a further three-way valve, connected to said fourth port of said four-way valve.

Still according to the invention, said device could comprise closing means, as a clamp and the like, arranged in one or more of said ports of said four-way valve, said closing means being preferably arranged on said first, second and fourth ports.

Advantageously according to the invention, said four-way valve comprises a three-way valve, whose ports are selectively opened or closed independently of the other, and a fourth port.

Further according to the invention, said filtering means comprise lysing, preloaded within said filtering syringe.

Always according to the invention, said filtering means comprise a filter for blood contamination interposed between said inlet/outlet mouth of said extraction syringe and said inlet/outlet mouth of said filtering syringe.

The present invention will be now described, for illustrative but not limitative purposes, according to its preferred embodiments, with particular reference to the figures of the enclosed drawings, wherein:
figure 1 shows the scheme of a first embodiment of an extraction and analysis device for synovial liquid according to the present invention;
figure 2 shows a part of a second embodiment of an extraction and analysis device according to the present invention; and
figure 3 shows the scheme of a third embodiment of an extraction and analysis device for synovial liquid according to the present invention.

In the various figures, similar parts will be indicated by the same reference numbers.

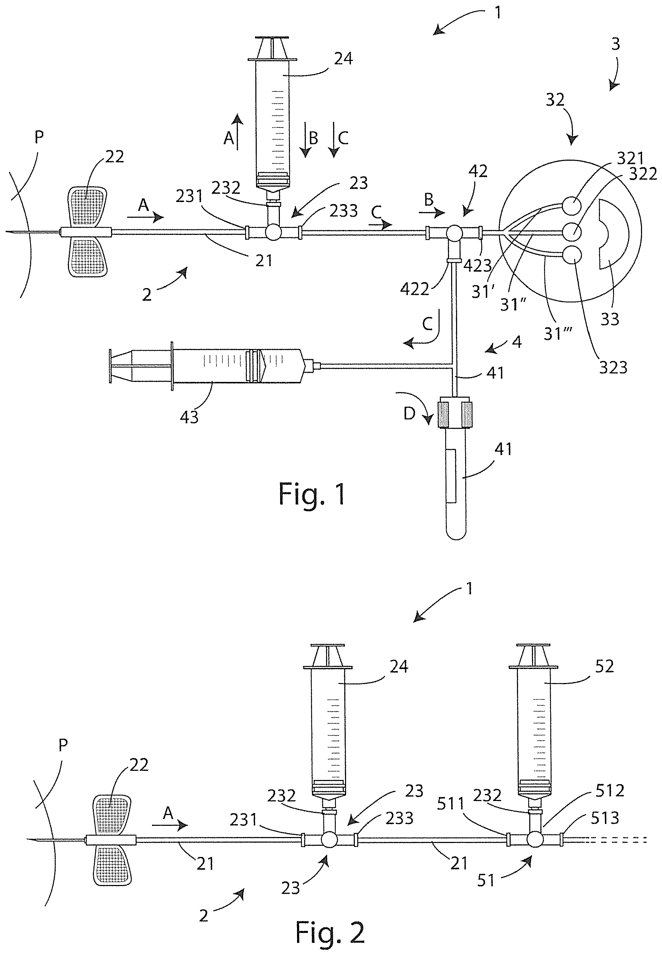

Referring to figure 1, an extraction and analysis device 1 for synovial liquid according to the present invention is seen. Naturally, the extraction and analysis device 1 can also be used for other anatomical tissues in liquid form.

The extraction and analysis device 1 essentially comprises a synovial liquid extraction line 2, an analysis unit 3, and a collecting line of the synovial liquid 4.

Said extraction line 2 comprises a extraction duct 21, a withdrawn needle 22, connected to said extraction duct 21, a first three-way valve 23, arranged along said extraction duct 21, and an extraction syringe 24 to allow the extraction of the synovial liquid from a patient P.

Said first three-way valve 23 has three ports, respectively a first port 231, fluid-dynamically connected, i.e. by means of the duct 21, with said withdrawn needle 22, a second port 232, connected with said extraction syringe 24, and a third port 233, whose connection is better defined in the following.

Ports 231, 232 and 233 of said three-way valve 23 are all selectively openable or closable independently from the other and allow the passage in input or output of a fluid, which, as said, in the present case is synovial fluid.

Said analysis unit 3 comprises three ducts 31', 31" and 31''', all connected to the end of said extraction duct 21, opposite to the one to which said withdrawn needle 22 is connected.

Said analysis unit 3 also includes an analysis device 32, having three receptacles 321, 322 and 323, in which respectively in three different reagents are arranged. In particular, esterase is placed in said first receptacle 321, CRP (C-Reactive Protein) is placed in said second receptacle 322 and Glucose is placed in said third receptacle 323.

The analysis device 32 also comprises a reading scale 33, the operation of which will be better described below.

The collecting line of the synovial fluid 4 comprises a collecting duct 41, having one end connected to a port of a second three-way valve 42, arranged on said extraction duct 21, of the same type of said first three-way valve 23 and arranged downstream of the latter.

Said second three-way valve 42 has a first port 421, connected with the section of the extraction duct 21 connected to said third port 233 of said first three-way valve 23, a second port 422, connected to said collecting duct 41, and a third port 423, connected to the section of the extraction duct 21 connected to said analysis device 3. Said three-way valve 42 is therefore arranged along said extraction duct 21, interposed between said first three-way valve 23 and said analysis device 32.

Said synovial liquid collecting line 4 also includes a vacutainer 44, removably connected to one end of said collecting duct 41.

Said vacutainer 44 is intended for collecting synovial fluid. Said synovial liquid collecting line 4 comprises also, in the present embodiment a suction syringe 43, connected to said collecting duct 41.

The operation of the extraction and analysis device 1 described above is as follows.

Once the extraction needle 22 is inserted, for example in an area of which verifying the possible presence of an infection is necessary, the third port 233 of said first three-way valve 23 is closed. In this way, the connection of the section of the extraction duct 21 between said first three-way valve 23 to said analysis device 32 or to the second three-way valve 42, is interrupted.

The ports 231 and 232 of said first three-way valve 23 are open. In this way, by acting on the extraction syringe 24, it is possible to extract the synovial fluid, collecting it in the reservoir of the extraction syringe 24 itself. The synovial fluid follows, with reference to figure 1, the arrow A.

Subsequently, the first door 231 is closed and the third door 233 opened. In addition, the first 421 and the third 423 port of the second three-way valve 42 are open, while the second port 422 remains closed. In this way, by pressing the plunger of the extraction syringe 24, gradually transferring at least part of the synovial fluid collected in said analysis device 3 is possible, following the arrow B. In this way, the synovial fluid is distributed on the three ducts 31', 31" and 31''' and on the three receptacles 321, 322 and 323, thus entering in contact with the three reagents. The reading scale 33 shows the chromatic variations of the three reagents, thus allowing to detect the degree of infection of the synovial fluid in real time. In this way, the surgeon or the health professional has a first clinical picture, to promptly intervene if necessary.

In particular, in the table 1 below the data for glucose, leukocyte esterase and CRP are shown, for two groups of patients, which do not differ significantly for sex or age. The first group includes 45 patients and they are aseptic; the second patients group B includes 15 patients and they are infected.

In patients group A, only one patient has had a positive result (shown as 1+) for leukocyte esterase, while in other patients the leukocyte esterase has shown results either negative (n = 51) or present in traces (n = 9).

In contrast, in patients with infection, the leukocyte esterase has been shown as negative in 2 patients, 1+ in 11 patients and 2+ in 8 patients. In group A, the glucose was negative in 4 patients, detected in traces, 1+, 2+, respectively for 14, 37, 6 patients, while, in the test group B, glucose was negative in 10 patients, present in traces in 6 patients and referred to as 1+ in 5 patients. Finally, the CRP was significantly higher in patients with infection compared to patients without infection (p <0,001).

**Table 1**

| | Group A | Group B |
|---|---|---|
| Leukocyte esterase (positive/negative) | 1/60 | 19/2 * |
| Glucose (positive/negative) | 43/18 | 5/16 ** |
| CRP (mg/L) | 7.92 ±20.0 | 40.3 ± 30.6*** |
| *p < 0.001 vs Group A; ** p=0.0016 vs Group A; ***p<0.0005 vs Group A | | |

The test of leukocyte esterase showed highest sensitivity and specificity values, followed in order by CRP and glucose as shown in Table 2 (sensitivity).

When the leukocyte esterase was combined with CRP, the sensitivity increased to 100%, while no differences were observed for leukocyte esterase combined with glucose. A sensitivity of 95,2% was observed when the CRP measures were combined with glucose test.

**Table 2**

| | Leukocyte esterase | Glucose | CRP |
|---|---|---|---|
| Sensitivity | 90.5% | 76.2% | 85, 7% |
| | (68.2 - 98.3) | (52.4 - 90.9) | (62.6 - 96.2) |
| Specificity | 98.4 % | 70.5 % | 88.5% |
| | (90-0 - 99.9) | (57.2 - 81.1) | (79.2 - 95.9) |
| Positive predictive value | 95.0 % | 47.0% | 72 % |
| | (73.0 - 99.7) | (30.1 - 64.6) | (50.4 - 87.1) |
| Negative predictive value | 96.8% | 89.6% | 94.7% |
| | (87.8 - 99.4) | (76.6 - 96.1) | (84.4 - 98.6) |

In case of collected additional synovial fluid were available in the extraction syringe 24 and if necessary, collecting said synovial fluid in a totally sterile way and send it to a laboratory for culture analysis is possible. For this purpose, it is sufficient to close said third port 423 and to open said first 421 and second 422 port of said second three-way valve 42. Subsequently, the plunger of the syringe extraction 24 is pressed and, by means of said suction syringe 43, the synovial fluid previously collected in the reservoir of said extraction syringe 24 is aspirated. The path of the synovial fluid is that of the arrow C. Finally, said second port 422 of said second three-way valve 42 is closed and the plunger of the suction syringe 43 is pressed, so as to convey the synovial fluid collected in vacutainer 44, according to arrow D.

It is considered that typically 5-10% of the synovial fluid extracted presents traces of blood, that make the color of the same tending to red. In such situations it is commonly said that the color changes to red. This implies that the reading and the detection of the esterase reagent, which is based on a red coloration of the esterase, can be not accurate or not reliable.

To reduce this problem the extraction device comprises means for filtering the plasma 5. Said means for filtering the plasma 5 comprise a third three-way valve 51, having the first port 511, connected to the section of the extraction duct 21 connected to said third 233 port of said first three-way valve 23 and the third port 513 connected to the section of extraction duct 21 connected to said first port 421 of said second three-way valve 42.

Said means for filtering the plasma 5 (see figure 2) comprise a supplementary circuit, also comprising a filtering syringe 52, connected to said second port 512 of said third three-way valve 51. Said filtering syringe 52 is, in the present embodiment, preloaded with lysing.

Properly opening the ports of said first three-way valve 23 and of said third three-way valve 51 of said plasma filtering means 5, the extracted synovial fluid and preliminarily collected in said extraction syringe 24 is collected into said syringe filtering 52, so as to mix it with said lysing reagent, thereby obtaining the desired filtering. Said synovial fluid thus collected is then transmitted to said analysis device 3.

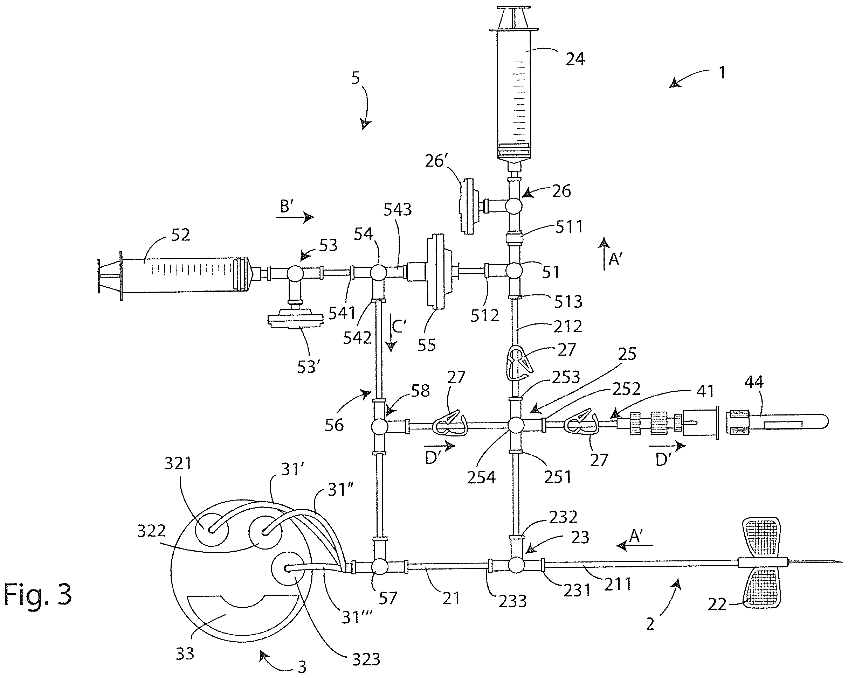

Figure 3 shows a further embodiment of the extraction and analysis device 1, wherein the extraction line 2 comprises a first duct 211, which connects the withdrawal needle 22 and the analysis unit 3, and a second duct 212, which connects the extraction syringe 24 to the first duct 211.

Said first three-way valve 23 is arranged along said first duct 211 and on second port 232 presents the second duct 212. Along the second duct 212 a four-way valve 25 is arranged, which is substantially constituted by a common three-way valve, such as those described above, and a further port, which is not selectively closable on the port, so as to be also selectively openable or closable by means of its three-way valve. Said four-way valve 25 has the first port 251 connected to said second port 232 of said first three-way valve 23, i.e. to said first duct 211. The second port 252 connected to said vacutainer 44 and the third port 253 connected to said syringe extraction 24. The fourth port 254, not selectively closable, is connected to a filtering line 56, that will be better said below.

To make the ports of the four-way valve 25 selective, safety clamps 27 are arranged on the ducts in correspondence of the doors, closing or opening which, it is possible to close or to open the ports of the valve itself.

In correspondence of said extraction syringe 24 and of said filtering syringe 52, both a three-way valve, indicated respectively with 26 and 53, as well as a respective air filter 26' and 53' are installed, properly selectable by means of the ports of the three-way valves 26 and 53.

Said plasma filtering means 5 are arranged in series between said second port 232 of said first three-way valve 23 and said four-way valve 25.

Furthermore, said filtering means comprise a fourth three-way valve 54, having the first port 541 connected to said filtering syringe 52 and the second port 542 connected to said filtering line 56, which connects, by means of a three-way valve 57, to an analysis unit 3 and to a further three-way valve 58, 254 to said fourth port of said four-way valve 25.

The filtering means also comprise a filter for blood contamination 55, connected to the second port 512 of said third three-way valve 51 of the plasma filter means 5.

When the synovial fluid is collected in said extraction syringe 24, following the arrow A', if necessary it is sent in said filtering syringe 52, which in this case can or cannot provide the preloaded lysing, according to the arrow B', so as to pass through the filter for blood contamination 55. Subsequently, the synovial fluid is transmitted to said analysis device 32, according to the arrow C', through filtering line 56, by the appropriate opening or closing of the valve ports 57 and 58 on the filtering line 56.

The synovial fluid present in the reservoir of the syringe 52 can then be re-collected in the vacutainer 44, flowing in the direction of arrow D', closing the first 251 and the third port 253 and closing the clamp 27 on the third port 253, and opening the second port 252 and the safety clamp 27 on said fourth port 254 and said second port 252.

Also in this case, the synovial fluid filtered from the blood traces, when arranged in contact with the three reagents, allows to detect the degree of the infection by colorimetric enzymatic test.

In any case, once collected into the syringe 24, the synovial fluid may be subsequently inserted directly into the vacutainer 44, in case of the with drawal were not entirely filtered or not filtered at all.

As it can be observed, the extraction and analysis device 1 is completely sterile and closed, in such a way that, once the synovial fluid is aspirated from the articulation, there is no of contamination of the liquid from the outside.

The use of clamps 27 allows the closure of the ducts on which they are arranged, by restriction . In particular, it is observed the clamp 27 arranged on the conduit 254 connected to the fourth port of said four-way valve 25, which allows the selective closure.

An advantage of the present invention is that of allowing a test on the synovial fluid pre-, intra- and post-operative, allowing in this way to obtain an immediate diagnosis (60-120 seconds) on the state of the articulation.

A further advantage of the present invention is to allow the real-time analysis of synovial fluid in a completely closed circuit way, allowing the withdrawal of the synovial fluid and its analysis in line, in such a way as to obtain a diagnosis in immediate times. The device is based on the use of different markers, such as leukocyte esterase and glucose, but it can be customizable by adding other markers according to the surveys to be carried out.

Another advantage of the present invention is that of allowing the possible collection of the synovial fluid by means of a vacuum system always, in line, in a test tube, vacutainer type or the like, which can be transported into the laboratory for cultural examination.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Device for extraction and analysis (1) of anatomical tissues, such as synovial liquid and the like, comprising
an extraction line (2, 21, 211, 212) of said tissue to be analyzed, having a needle (22), for the withdrawal of said tissue to be analyzed, an extraction duct (21, 211, 212), an extraction syringe (24), having an inlet/outlet mouth connected fluid-dynamically to said needle (22) by means of said extraction duct (21, 211, 212), and
an analysis device (3), fluid-dynamically connected to said inlet/outlet mouth of said extraction syringe (24) by means of said extraction duct (21, 211, 212), said analysis device (3) having one or more receptacles (321, 322, 323), in each of which a respective reagent is arranged, and reading means (33), for detecting possible alterations of said reagents, said one or more receptacles (321, 322, 323) comprising a first receptacle (321), into which a first reagent is placed,
wherein said extraction duct (21, 211, 212) of said extraction line (2) comprises a first three-way valve (23), having a first port (231), connected to said withdrawal needle (22), a second port (232), connected to said inlet/outlet mouth of said extraction syringe (24), and a third port (233) connectable to said analysis device (3), each of said ports (231, 232, 233) being selectively openable or closable independently of the other, **characterised in that** said one or more receptacles (321, 322, 323) comprise a second receptacle (322), into which a the second reagent is placed, and a third receptacle (323), into which a third reagent is placed.

2. Device (1) according to claim 1, **characterized in that** said first reagent is esterases, said second reagent is CRP (C-Reactive Protein) and said third reagent is glucose.

3. Device (1) according to any one of the preceding claims, **characterized in that** said reading means comprise a reading scale (33), capable to show the color variations of said reagents, allowing to detect the degree of infection of said anatomical tissue, as synovial liquid and the like.

4. Device (1) according to any one of the preceding claims, **characterized in that** said extraction line (2) comprises air filtering means, in their turn comprising
an air filter (26') and
a respective three-way valve (26), having three ports selectively openable or closable independently of the other, one of said three ports being connected to said inlet/outlet mouth of said extraction syringe (24), and another of said three ports being connected to said air filter (26').

5. Device (1) according to any one of the preceding claims, **characterized in that** said device (1) also comprises an anatomical tissues collecting line (4), having a vacutainer (44), fluid-dynamically connectable to the inlet/outlet mouth of a suction syringe (43).

6. Device (1) according to the preceding claim, **characterized in that** said collecting line comprises said suction syringe (43) having an inlet/outlet mouth fluid-dynamically connected to said inlet/outlet mouth of said extraction syringe (24).

7. Device (1) according to claim 6, **characterized in that** said collecting line (4) comprises a second three-way valve (42), having a first port (421), fluid-dynamically connected to said third port (233) of said first three-way valve (23) along said extraction duct (21, 211, 212) of said extraction line (2), a second port (422) fluid-dynamically connected to said inlet/outlet mouth of said suction syringe (43), and a third port (423), fluid-dynamically connected to said analysis device (3), each of said ports (421, 422, 423) being selectively openable or closable independently of the other.

8. Device (1) according to claim 7, **characterized in that** said collecting line (4) comprises filtering means comprising
an air filter and
a respective three-way valve, having three ports selectively openable or closable independently of the other, one of said three ports being connected to said inlet/outlet mouth of said suction syringe (43), and another one of said three ports being connected to said air filter.

9. Device (1) according to any one of the preceding claims, **characterized in that** it comprises plasma filtering means (5), adapted to filter the plasma from the said tissue to be analyzed, arranged between said inlet/outlet mouth of said extraction syringe (24) and said analysis device (3).

10. Device (1) according to claim 9, **characterized in that** said plasma filtering means (5) comprise a filtering syringe (52), having an inlet/outlet mouth, a third three-way valve (51), having a first port (511), fluid-dynamically connected to said inlet/outlet mouth of said extraction syringe (24), a second port (512), to which the inlet/outlet mouth of said filtering syringe (52) is connected, and a third port (513), fluid-dynamically and selectively connected to said analysis device (3).

11. Device (1) according to claim 10, **characterized**
**in that** said extraction line (2) comprises a first duct (211), which connects said withdrawal needle (22) and the analysis unit (3), said first three-way valve (23) being arranged along said first duct (211), and a second duct (212), which connects the extraction syringe (24) to said first duct (211), a four-way valve (25) having a first port (251) fluid-dynamically connected to said withdrawal needle (22), a second port (252) fluid-dynamically connectable to said vacutainer (44), a third port (253) fluid-dynamically connectable to said extraction syringe (24) and a fourth port (254), each of said ports (251, 252, 253, 254) being selectively opened or closed independently of the other, and
**in that** said filtering means (5) comprises a filtering line (56), fluid-dynamically connected between said filtering syringe (52) and said analysis unit (3), and a further three-way valve (58), connected to said fourth port (254) of said four-way valve (25).

12. Device (1) according to claim 11, **characterized in that** it comprises closing means (26), as a clamp and the like, arranged in one or more of said ports (251, 252, 253, 254) of said four-way valve (25), said closing means (26) being preferably arranged on said first (251), second (252) and fourth (254) ports.

13. Device (1) according to any one of claims 11 or 12, **characterized in that** said four-way valve comprises a three-way valve, whose ports (251, 252, 253) are selectively openable or closable independently of the other, and a fourth port (254).

14. Device (1) according to any one of claims 10-13, **characterized in that** said filtering means comprise lysing, preloaded within said filtering syringe (52).

15. Device (1) according to any one of claims 10-14, **characterized in that** said filtering means comprise a filter for blood contamination (55) interposed between said inlet/outlet mouth of said extraction syringe (24) and said inlet/outlet mouth of said filtering syringe (52).

## Patentansprüche

1. Vorrichtung zur Extraktion und Analyse (1) anatomischer Gewebe, beispielsweise Synovialflüssigkeit und dergleichen, umfassend
eine Extraktionsleitung (2, 21, 211, 212) des zu analysierenden Gewebes mit einer Nadel (22) zum Zurückziehen des zu analysierenden Gewebes, ein Extraktionsrohr (21, 211, 212), eine Extraktionsspritze (24) mit einem Einlass-/Auslassmund, die mittels des Extraktionsrohrs (21, 211, 212) fluiddynamisch mit der Nadel (22) verbunden ist, und
eine Analysevorrichtung (3), die mittels des Extraktionsrohrs (21, 211, 212) fluiddynamisch mit dem Einlass-/Auslassmund der Extraktionsspritze (24) verbunden ist, wobei die Analysevorrichtung (3) ein oder mehrere Behältnisse (321, 322, 323), in denen jeweils ein entsprechendes Reagens angeordnet ist, und Ablesemittel (33) zum Detektieren möglicher Veränderungen der Reagenzien aufweist, wobei das eine oder die mehreren Behältnisse (321, 322, 323) ein erstes Behältnis (321) umfassen, in das ein erstes Reagens platziert wird,
wobei das Extraktionsrohr (21, 211, 212) der Extraktionsleitung (2) ein erstes Dreiwegeventil (23) umfasst, das eine erste Öffnung (231), die mit der Rückziehnadel (22) verbunden ist, eine zweite Öffnung (232), die mit dem Einlass-/Auslassmund der Extraktionsspritze (24) verbunden ist, und eine dritte Öffnung (233), die mit der Analysevorrichtung (3) verbindbar ist, aufweist, wobei jede der Öffnungen (231, 232, 233) unabhängig voneinander selektiv geöffnet oder geschlossen werden kann,
**dadurch gekennzeichnet, dass** das eine oder die mehreren Behältnisse (321, 322, 323) ein zweites Behältnis (322), in das ein zweites Reagens platziert wird, und ein drittes Behältnis (323), in das ein drittes Reagens platziert wird, umfassen.

2. Vorrichtung (1) nach Anspruch 1, **dadurch gekennzeichnet, dass** das erste Reagens Esterasen ist, das zweite Reagens CRP (C-reaktives Protein) ist und das dritte Reagens Glucose ist.

3. Vorrichtung (1) nach einem der vorstehenden Ansprüche, **dadurch gekennzeichnet, dass** die Ablesemittel eine Ableseskala (33) umfassen, die die Farbvariationen der Reagenzien zeigen kann, was das Detektieren des Infektionsgrades des anatomischen Gewebes, beispielsweise Synovialflüssigkeit und dergleichen, zulässt.

4. Vorrichtung (1) nach einem der vorstehenden Ansprüche, **dadurch gekennzeichnet, dass** die Extraktionsleitung (2) Luftfiltriermittel umfasst, die wiederum Folgendes umfassen
einen Luftfilter (26') und
ein entsprechendes Dreiwegeventil (26) mit drei Öffnungen, die unabhängig voneinander selektiv geöffnet oder geschlossen werden können, wobei eine der drei Öffnungen mit dem Einlass-/Auslassmund der Extraktionsspritze (24) verbunden ist und eine andere der drei Öffnungen mit dem Luftfilter (26') verbunden ist.

5. Vorrichtung (1) nach einem der vorstehenden Ansprüche, **dadurch gekennzeichnet, dass** die Vorrichtung (1) zudem eine Sammelleitung (4) für anatomische Gewebe mit einem Blutentnahmeröhrchen (44), das fluiddynamisch mit dem Einlass-/Auslassmund einer Saugspritze (43) verbindbar ist, umfasst.

6. Vorrichtung (1) nach einem der vorstehenden Ansprüche, **dadurch gekennzeichnet, dass** die Sammelleitung die Saugspritze (43) mit einem Einlass-/Auslassmund, der fluiddynamisch mit dem Einlass-/Auslassmund der Extraktionsspritze (24) verbunden ist, umfasst.

7. Vorrichtung (1) nach Anspruch 6, **dadurch gekennzeichnet, dass** die Sammelleitung (4) ein zweites Dreiwegeventil (42) umfasst, das eine erste Öffnung (421), die fluiddynamisch mit der dritten Öffnung (233) des ersten Dreiwegeventils (23) entlang des Extraktionsrohrs (21, 211, 212) der Extraktionsleitung (2) verbunden ist, eine zweite Öffnung (422), die fluiddynamisch mit dem Einlass-/Auslassmund der Ansaugspritze (43) verbunden ist, und eine dritte Öffnung (423), die fluiddynamisch mit der Analysevorrichtung (3) verbunden ist, aufweist, wobei jede der Öffnungen (421, 422, 423) unabhängig voneinander selektiv geöffnet oder geschlossen werden kann.

8. Vorrichtung (1) nach Anspruch 7, **dadurch gekennzeichnet, dass** die Sammelleitung (4) Filtriermittel umfasst, die Folgendes umfassen
einen Luftfilter und
ein entsprechendes Dreiwegeventil mit drei Öffnungen, die unabhängig voneinander selektiv geöffnet oder geschlossen werden können, wobei eine der drei Öffnungen mit dem Einlass-/Auslassmund der Ansaugspritze (43) verbunden ist und eine andere der drei Öffnungen mit dem Luftfilter verbunden ist.

9. Vorrichtung (1) nach einem der vorstehenden Ansprüche, **dadurch gekennzeichnet, dass** sie Plasmafiltriermittel (5) umfasst, die angepasst sind, um das Plasma aus dem zu analysierenden Gewebe zu filtrieren, angeordnet zwischen dem Einlass-/Auslassmund der Extraktionsspritze (24) und der Analysevorrichtung (3).

10. Vorrichtung (1) nach Anspruch 9, **dadurch gekennzeichnet, dass** die Plasmafiltriermittel (5) Folgendes umfassen: eine Filtrierspritze (52) mit einem Einlass-/Auslassmund, ein drittes Dreiwegeventil (51), das eine erste Öffnung (511), die fluiddynamisch mit dem Einlass-/Auslassmund der Extraktionsspritze (24) verbunden ist, eine zweite Öffnung (512), mit der der Einlass-/Auslassmund der Filtrierspritze (52) verbunden ist, und eine dritte Öffnung (513), die fluiddynamisch und selektiv mit der Analysevorrichtung (3) verbunden ist, aufweist.

11. Vorrichtung (1) nach Anspruch 10, **dadurch gekennzeichnet,**
**dass** die Extraktionsleitung (2) Folgendes umfasst: ein erstes Rohr (211), das die Rückziehnadel (22) und die Analyseeinheit (3) verbindet, wobei das erste Dreiwegeventil (23) entlang des ersten Rohrs (211) angeordnet ist, und ein zweites Rohr (212), das die Extraktionsspritze (24) mit dem ersten Rohr (211) verbindet, ein Vierwegeventil (25), das eine erste Öffnung (251), die fluiddynamisch mit der Rückziehnadel (22) verbunden ist, eine zweite Öffnung (252), die fluiddynamisch mit dem Blutentnahmeröhrchen (44) verbindbar ist, eine dritte Öffnung (253), die fluiddynamisch mit der Extraktionsspritze (24) verbindbar ist, und eine vierte Öffnung (254) aufweist, wobei jede der Öffnungen (251, 252, 253, 254) unabhängig voneinander selektiv geöffnet und geschlossen wird, und
**dass** das Filtriermittel (5) eine Filtrierleitung (56), die fluiddynamisch zwischen der Filtrierspritze (52) und der Analyseeinheit (3) verbunden ist, und ein weiteres Dreiwegeventil (58), das mit der vierten Öffnung (254) des Vierwegeventils (25) verbunden ist, umfasst.

12. Vorrichtung (1) nach Anspruch 11, **dadurch gekennzeichnet, dass** sie ein Schließmittel (26), beispielsweise eine Klemme und dergleichen, umfasst, angeordnet in einer oder mehreren der Öffnungen (251, 252, 253, 254) des Vierwegeventils (25), wobei das Schließmittel (26) bevorzugt an der ersten (251), zweiten (252) und vierten (254) Öffnung angeordnet ist.

13. Vorrichtung (1) nach einem der Ansprüche 11 oder 12, **dadurch gekennzeichnet, dass** das Vierwegeventil ein Dreiwegeventil, dessen Öffnungen (251, 252, 253) unabhängig voneinander selektiv geöffnet oder geschlossen werden können, und eine vierte Öffnung (254) umfasst.

14. Vorrichtung (1) nach einem der Ansprüche 10-13, **dadurch gekennzeichnet, dass** die Filtriermittel innerhalb der Filtrierspritze (52) vorgeladenes Lysemittel umfassen.

15. Vorrichtung (1) nach einem der Ansprüche 10-14, **dadurch gekennzeichnet, dass** die Filtriermittel einen Blutkontaminierungsfilter (55) umfassen, der zwischen dem Einlass-/Auslassmund der Extraktionsspritze (24) und dem Einlass-/Auslassmund der Filtrierspritze (52) geschaltet ist.

## Revendications

1. Dispositif pour l'extraction et l'analyse (1) de tissus anatomiques, tels que du liquide synovial et similaire, comprenant
une tubulure d'extraction (2, 21, 211, 212) dudit tissu à analyser, ayant une aiguille (22), pour le retrait dudit tissu à analyser, un conduit d'extraction (21, 211, 212), une seringue d'extraction (24), ayant une embouchure d'entrée/sortie raccordée avec une dynamique de fluide à ladite aiguille (22) au moyen dudit conduit d'extraction (21, 211, 212), et
un dispositif d'analyse (3), raccordé avec une dynamique de fluide à ladite embouchure d'entrée/sortie de ladite seringue d'extraction (24) au moyen dudit conduit d'extraction (21, 211, 212), ledit dispositif d'analyse (3) ayant un ou plusieurs réceptacles (321, 322, 323), dans chacun desquels un réactif respectif est agencé, et des moyens de lecture (33), pour détecter des altérations possibles desdits réactifs, lesdits un ou plusieurs réceptacles (321, 322, 323) comprenant un premier réceptacle (321), dans lequel un premier réactif est placé, dans lequel ledit conduit d'extraction (21, 211, 212) de ladite tubulure d'extraction (2) comprend une première valve à trois voies (23), ayant un premier orifice (231), raccordé à ladite aiguille de retrait (22), un deuxième orifice (232), raccordé à ladite embouchure d'entrée/sortie de ladite seringue d'extraction (24), et un troisième orifice (233) qui peut être raccordé audit dispositif d'analyse (3), chacun desdits orifices (231, 232, 233) étant ouvrable ou refermable sélectivement indépendamment des autres,
**caractérisé en ce que** lesdits un ou plusieurs réceptacles (321, 322, 323) comprennent un deuxième réceptacle (322), dans lequel un deuxième réactif est placé, et un troisième réceptacle (323), dans lequel un troisième réactif est placé.

2. Dispositif (1) selon la revendication 1, **caractérisé en ce que** ledit premier réactif est les estérases, ledit deuxième réactif est la CRP (protéine c réactive) et ledit troisième réactif est le glucose.

3. Dispositif (1) selon l'une quelconque des revendications précédentes, **caractérisé en ce que** lesdits moyens de lecture comprennent une échelle de lecture (33), capable de montrer les variations de couleur desdits réactifs, permettant de détecter le degré d'infection dudit tissu anatomique, en tant que liquide synovial et similaire.

4. Dispositif (1) selon l'une quelconque des revendications précédentes, **caractérisé en ce que** ladite tubulure d'extraction (2) comprend des moyens de filtrage d'air, comprenant à leur tour
un filtre à air (26'), et
une valve à trois voies respective (26), ayant trois orifices ouvrables et refermables sélectivement indépendamment des autres, l'un desdits trois orifices étant raccordé à ladite embouchure d'entrée/sortie de ladite seringue d'extraction (24), et un autre desdits trois orifices étant raccordé audit filtre à air (26').

5. Dispositif (1) selon l'une quelconque des revendications précédentes, **caractérisé en ce que** ledit dispositif (1) comprend également une tubulure de collecte de tissus anatomiques (4), ayant un vacutainer (44), qui peut être raccordé avec une dynamique de fluide à l'embouchure d'entrée/sortie d'une seringue d'aspiration (43).

6. Dispositif (1) selon la revendication précédente, **caractérisé en ce que** ladite tubulure de collecte comprend ladite seringue d'aspiration (43) ayant une embouchure d'entrée/sortie raccordée avec une dynamique de fluide à ladite embouchure d'entrée/sortie de ladite seringue d'extraction (24).

7. Dispositif (1) selon la revendication 6, **caractérisé en ce que** ladite tubulure de collecte (4) comprend une deuxième valve à trois voies (42), ayant un premier orifice (421), raccordé avec une dynamique de fluide audit troisième orifice (233) de ladite première valve à trois voies (23) le long dudit conduit d'extraction (21, 211, 212) de ladite tubulure d'extraction (2), un deuxième orifice (422) raccordé avec une dynamique de fluide à ladite embouchure d'entrée/sortie de ladite seringue d'aspiration (43), et un troisième orifice (423), raccordé avec une dynamique de fluide audit dispositif d'analyse (3), chacun desdits orifices (421, 422, 423) étant ouvrable ou refermable sélectivement indépendamment des autres.

8. Dispositif (1) selon la revendication 7, **caractérisé en ce que** ladite tubulure de collecte (4) comprend des moyens de filtrage comprenant
un filtre à air et
une valve à trois voies respective, ayant trois orifices ouvrables ou refermables sélectivement indépendamment des autres, l'un desdits trois orifices étant raccordé à ladite embouchure d'entrée/sortie de ladite seringue d'aspiration (43), et un autre desdits trois orifices étant raccordé audit filtre à air.

9. Dispositif (1) selon l'une quelconque des revendications précédentes, **caractérisé en ce qu'**il comprend des moyens de filtrage de plasma (5), adaptés pour filtrer le plasma provenant dudit tissu à analyser, agencés entre ladite embouchure d'entrée/sortie de ladite seringue d'extraction (24) et ledit dispositif d'analyse (3).

10. Dispositif (1) selon la revendication 9, **caractérisé en ce que** lesdits moyens de filtrage de plasma (5) comprennent une seringue de filtrage (52), ayant une embouchure d'entrée/sortie, une troisième valve à trois voies (51), ayant un premier orifice (511), raccordé avec une dynamique de fluide à ladite embouchure d'entrée/sortie de ladite seringue d'extraction (24), un deuxième orifice (512), auquel l'embouchure d'entrée/sortie de ladite seringue d'extraction (52) est raccordée, et un troisième orifice (513), raccordé avec une dynamique de fluide et sélectivement audit dispositif d'analyse (3).

11. Dispositif (1) selon la revendication 10, **caractérisé**
**en ce que** ladite tubulure d'extraction (2) comprend un premier conduit (211), qui raccorde ladite aiguille de retrait (22) et l'unité d'analyse (3), ladite valve à trois voies (23) étant agencée le long dudit premier conduit (211), et un second conduit (212), qui raccorde la seringue d'extraction (24) audit premier conduit (211), une valve à quatre voies (25) ayant un premier orifice (251) raccordé avec une dynamique de fluide à ladite aiguille de retrait (22), un deuxième orifice (252) qui peut être raccordé avec une dynamique de fluide audit vacutainer (44), un troisième orifice (253) qui peut être raccordé avec une dynamique de fluide à ladite seringue d'extraction (24) et un quatrième orifice (254), chacun desdits orifices (251, 252, 253, 254) étant ouvert ou fermé sélectivement indépendamment des autres, et
**en ce que** lesdits moyens de filtrage (5) comprennent une tubulure de filtrage (56), raccordée avec une dynamique de fluide entre ladite seringue de filtrage (52) et ladite unité d'analyse (3), et une valve à trois voies supplémentaire (58), raccordée audit quatrième orifice (254) de ladite valve à quatre voies (25).

12. Dispositif (1) selon la revendication 11, **caractérisé en ce qu'**il comprend des moyens de fermeture (26), en tant que pince et similaire, agencés dans un ou plusieurs desdits orifices (251, 252, 253, 254) de ladite valve à quatre voies (25), lesdits moyens de fermeture (26) étant de préférence agencés sur lesdits premier (251), deuxième (252) et quatrième (254) orifices.

13. Dispositif (1) selon l'une quelconque des revendications 11 ou 12, **caractérisé en ce que** ladite valve à quatre voies comprend une valve à trois voies, dont les orifices (251, 252, 253) sont ouvrables ou refermables sélectivement indépendamment des autres, et un quatrième orifice (254).

14. Dispositif (1) selon l'une quelconque des revendications 10 à 13, **caractérisé en ce que** lesdits moyens de filtrage comprennent une lyse, préchargée au sein de ladite seringue de filtrage (52).

15. Dispositif (1) selon l'une quelconque des revendications 10 à 14, **caractérisé en ce que** lesdits moyens de filtrage comprennent un filtre pour une contamination sanguine (55) interposé entre ladite embouchure d'entrée/sortie de ladite seringue d'extraction (24) et ladite embouchure d'entrée/sortie de ladite seringue de filtrage (52).
